Europäisches Patentamt

**European Patent Office**

**Office européen des brevets**

(19)

(11) Publication number:

**0 119 083**

**A2**

(12)

# EUROPEAN PATENT APPLICATION

(21) Application number: **84301619.7**

(22) Date of filing: **09.03.84**

(51) Int. Cl.³: **C 12 N 15/00**
**C 12 N 1/20**
**//(C12N1/20, C12R1/19)**

(30) Priority: **09.03.83 US 473548**

(43) Date of publication of application:
**19.09.84 Bulletin 84/38**

(84) Designated Contracting States:
**AT BE CH DE FR GB IT LI LU NL SE**

(71) Applicant: **GENEX CORPORATION**
**6110 Executive Boulevard**
**Rockville Maryland 20852(US)**

(72) Inventor: **Randall, Alan Roehl**
**13215 Country Ridge Drive**
**Germantown Maryland 20874(US)**

(72) Inventor: **Guyer, Mark Samuel**
**5710 Roosevelt**
**Bethesda Maryland 20817(US)**

(74) Representative: **Holmes, Michael John et al,**
**Frank B. Dehn & Co. Imperial House 15-19 Kingsway**
**London WC2B 6UZ(GB)**

(54) Novel plasmid cloning vectors.

(57) Disclosed is a series of hybrid bacterial plasmids which are useful for gene cloning in a wide variety of bacterial hosts. Also disclosed are microorganisms transformed by these plasmids.

EP 0 119 083 A2

Croydon Printing Company Ltd.

# NOVEL PLASMID CLONING VECTORS

## Technical Field

The present invention relates to bacterial plasmids. More particularly, the invention relates to hybrid plasmids that are useful for cloning genes in a variety of bacterial hosts.

## Background of the Invention

A number of excellent plasmid vectors have been developed in recent years for cloning genes in Escherichia coli. A well-known plasmid is pBR322, a small, multi-copy plasmid, which carries genes for determining resistance to both tetracycline and β-lactam antibiotics such as ampicillin. Although this plasmid has been used extensively for cloning and expressing various genes in E.coli, the fact that it normally replicates only in that bacterial species and closely related species has limited its usefulness for cloning and expressing particular genes that require a different host cell genetics or physiology.

Plasmids comparable to pBR322 for cloning genes into other types of bacteria, such as Pseudomonas, Rhizobium, and Rhodopseudomonas have not been readily available. Soil bacteria have the ability to degrade or modify a wide range of compounds and some species produce disease in animals and plants. As many activities of these bacteria are of medical, environmental, and commercial interest and importance, it would be valuable to be able to carry out genetic manipulations of the DNA of the organisms.

There have been reports of hybrid plasmids which can be used as cloning vectors in Pseudomonas. Two reports disclose hybrid plasmids composed of pBR322 and the broad host range plasmid RSF1010. (Bagdasarian, M. et al. Plasmids of Medical, Environmental and Commercial Importance (1979) Elsevier, North Holland Biomedical Press; Wood, D. et al. J. Bacteriology 14:1448-1451 (1981)). This latter plasmid confers resistance to streptomycin and sulfonamide and, like pBR322, replicates in the cell as a multi-copy plasmid. In contrast to pBR322, RSF1010 has relatively few convenient, unique restriction sites useful for cloning genes.

The hybrid plasmids disclosed by Wood et al. are composed of complete copies of pBR322 and RSF1010. The hybrid plasmids constructed by Bagdasarian et al. are composed of complete copies of pBR322 and nearly complete copies of RSF1010. The hybrid plasmids thus are structurally very similar; the primary difference between them is in their site of attachment. Bagdasarian et al. stated that the hybrid plasmid they disclosed was unable to transform Pseudomonas; Wood et al. demonstrated transfer and maintenance of their plasmid in Pseudomonas. As each of these hybrids have complete copies of pBR322 and complete or nearly complete copies of RSF1010 they have a number of duplicate restriction sites which were formed by the combination of the two plasmids. These duplicate sites limit the usefulness of the hybrid plasmid for insertion of cloned DNA.

A need exists for plasmid cloning vectors which have a high degree of versatility and can be used for cloning genes into a variety of bacterial hosts.

Disclosure of the Invention

In accordance with the present invention there is disclosed a series of hybrid plasmids which contain the wide host-range properties of RSF1010, replicate as multiple copy number plasmids, and express useful antibiotic resistances. One such hybrid plasmid contains DNA from the transposable element Tn9 and is characterized by the antibiotic resistance markers for chloramphenicol, streptomycin, and sulfonamide. Two plasmids contain DNA from the transposable element Tn9 and the tetracycline resistance gene from pBR322. Two other plasmids of this invention contain the ampicillin and tetracycline resistances of pBR322. These hybrid plasmids may be used for gene cloning in a wide variety of bacterial hosts.

In each case, the DNA from RSF 1010 present in the hybrid lacks at least the EcoRI restriction site.

According to the present invention, therefore, we provide a novel hybrid plasmid consisting of DNA from plasmid RF 1010 together with the DNA of the transposon Tn9 and/or the DNA of plasmid pBR 322, the DNA from plasmid RF 1010 lacking at least the EcoRI restriction site of the original plasmid.

This new series of plasmids is constructed using conventional genetic engineering techniques. The plasmids pBR322 and RSF1010, or derivatives thereof and in some instances pMS2 (the source of Tn 9), are digested with restriction enzymes, and the resulting DNA fragments are ligated, for example, with the enzyme T4 DNA ligase. The rejoined hybrid plasmids then may be introduced into a microorganism by transformation and selected by expression of the desired antibiotic resistances.

In one embodiment of this invention, the initial step in this process is to modify the RSF1010 plasmid. The plasmid has a single EcoRI site. Digesting the plasmid with EcoRI thus cuts the circle at one site, linearizing the plasmid. The linear DNA then is treated with the enzyme DNA polymerase in the presence of two deoxyribo nucleotides, a process which causes the single-stranded sticky ends to be filled, creating double-stranded blunt ends. The ends of the opened plasmid then are rejoined.

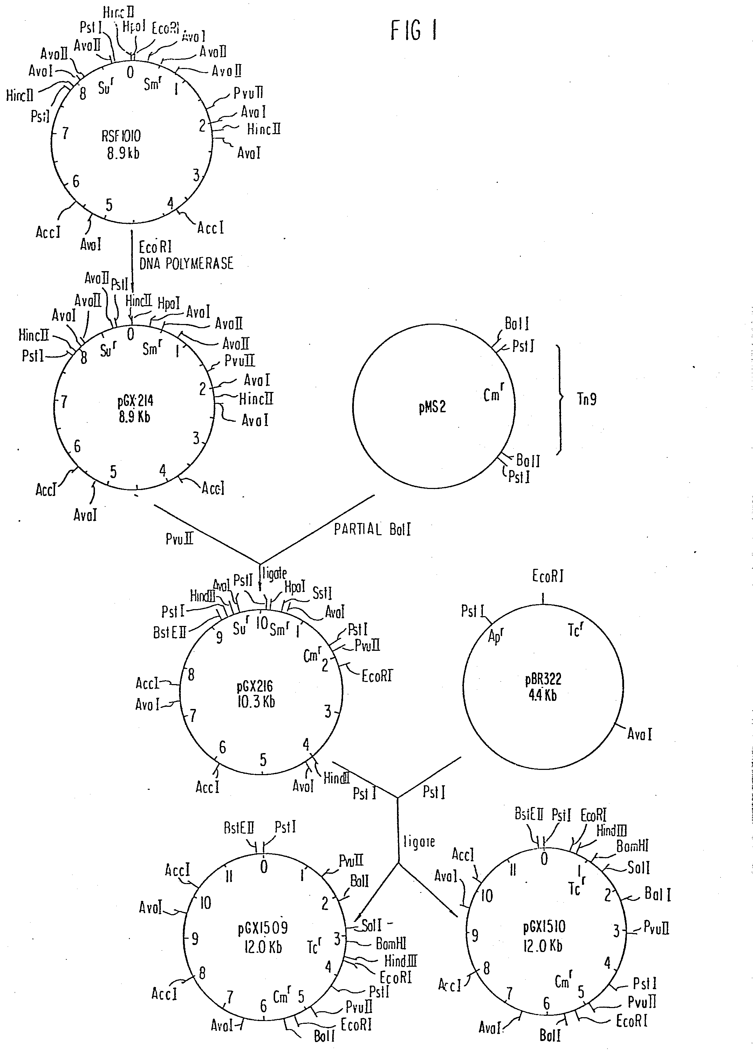

This action causes the alteration and, therefore, the loss of the EcoRI site on the plasmid. This modified plasmid has been designated pGX214. See Figure 1. of the accompanying drawings.

This new plasmid then can be modified by the insertion of a short segment of DNA derived from the transposon Tn9. The transposable element Tn9 contains a region of DNA which specifies resistance to the antibiotic chloramphenicol and is bordered by flanking regions of direct repeat DNA sequences. These sequences ordinarily facilitate in vivo excision and integration of the intact Tn9 element and thus permit it to transpose from one DNA site to another under certain circumstances.

Plasmid pMS2 contains the Tn9 element which is flanked on each side by DNA sequences which are recognized by the restriction enzymes BalI and PstI. (See Figure 1.) A portion of the Tn9 fragment can be cleaved from this plasmid by partial digestion of the plasmid with BalI. The DNA fragment removed from the plasmid then can be inserted into pGX214. The plasmid can be cleaved with the restriction enzyme PvuII which recognizes one site on the pGX214 plasmid; then the Tn9 DNA fragment can be ligated into the opened plasmid. The resulting plasmid has been designated pGX216. E.coli cells transformed with this new hybrid plasmid show resistance to streptomycin and chloramphenicol. An E.coli cell culture transformed with pGX 216 has been designated GX3607 (pGX216) and deposited with the Northern Regional Research Laboratory, Peoria, Illinois as NRRL B-15330.

Plasmid pGX216 contains three PstI recognition sites and pBR322 contains one such recognition site. By digesting each of the plasmids with PstI, the pGX216 plasmid can be cut and linearized into three fragments, and the pBR322 plasmid can be opened into a linear strand of DNA. The ends of the opened pBR322 plasmid then can be ligated to the ends of the pGX216 DNA segments. Composite plasmids can be transformed into E.coli and transformants selected that are resistant to chloramphenicol and streptomycin. Two different plasmids were isolated that contain the large PstI fragment from pGX216 and all of pBR322. These hybrid plasmids comprise pBR322 and the large fragment of pGX216, joined at their PstI sites. The two hybrid plasmids differ from one another in their orientation of the linear pBR322. These hybrid plasmids, shown in Figure 1, have been designated pGX1509 and pGX1510. In pGX1509, the orientation of the linear pBR322 is such that the EcoRI recognition site and the tetracycline resistance marker of the plasmid are located proximal to the chloramphenicol resistance gene of the Tn9 DNA sequence of the pGX216 fragment. In pGX1510, this end of the opened pBR322 plasmid is distal to the chloramphenicol resistance gene of the pGX216 fragment.

The pGX1509 and pGX1510 plasmids can be used as cloning vectors for a variety of procaryotic and eucaryotic genes in a wide range of hosts. An E.coli cell culture transformed with pGX1509 has been designated GX3601 (pGX1509) and has been deposited with the NRRL as NRRL B-15328. An E.coli cell culture transformed with pGX1510 has been designated GX3602 (pGX1510) and has been deposited as NRRL B-15329.

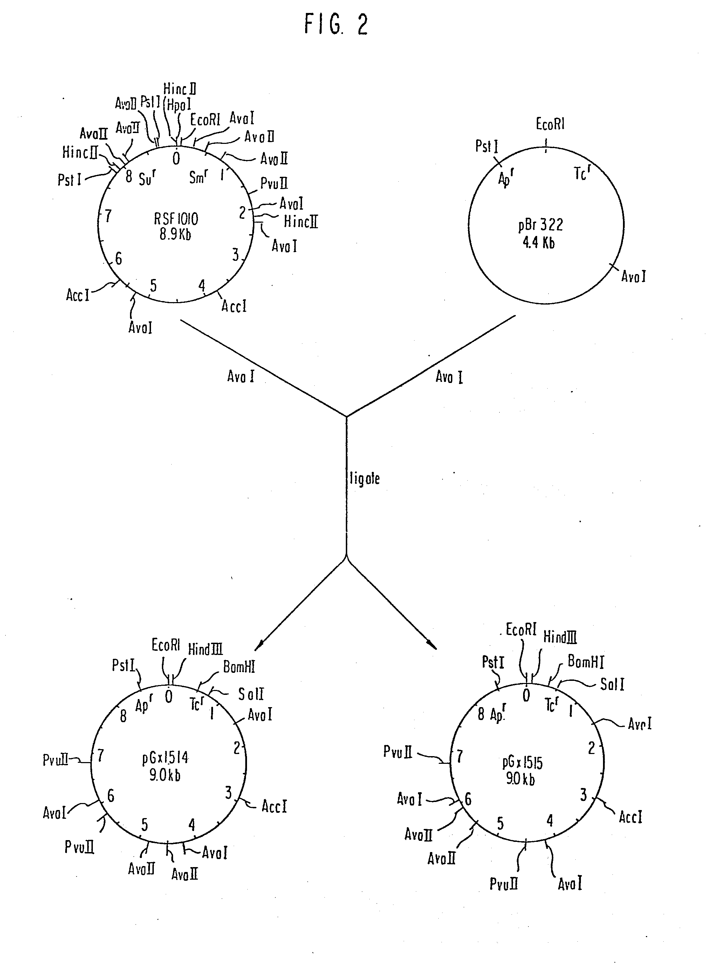

In another embodiment of this invention, novel hybrid plasmids can be formed by ligating fragments of RSF1010 directly into pBR322. RSF1010 contains multiple recognition sites for the restriction enzyme AvaI, and pBR322 contains a single site for this enzyme. The two plasmids can be digested with AvaI, mixed, and ligated. See Figure 2. A ligation mixture was used to transform Pseudomonas aeruginosa. Two hybrid plasmids have been isolated from the transformants and have been designated pGX1514 and pGX1515. The two plasmids differ from one another in the orientation of one of the fragments from RSF1010. In both plasmids a large RSF1010 fragment is ligated directly to the end of the opened pBR322 plasmid, proximal to the tetracycline resistance gene of pBR322. To this RSF1010 AvaI fragment is ligated a second, smaller RSF1010 fragment which contains a PvuII restriction enzyme site near one end of the fragment. In pGX1514 this second RSF1010 fragment is oriented such that the PvuII site is proximal to the PvuII site in the pBR322 segment. In pGX1515 the PvuII site is distal to the PvuII site in the pBR322 segment.

An E.coli cell culture transformed with pGX1514 has been designated GX3639 (pGX1514) and deposited with the NRRL as NRRL B-15331. An E.coli cell culture transformed with pGX1515 has been designated Gx3640 (pGX1515) and deposited as NRRL B-15332.

. The series of plasmids disclosed herein differ from the hybrids previously disclosed in the literature in several respects. Whereas the earlier plasmids are hybrids of pBR322 and complete or nearly complete RSF1010 and thus have a number of duplicated restriction sites, the pGX1514 and pGX1515 plasmids of the present invention preserve a number of important restriction sites, such as PstI and EcoRI, as unique restriction sites. This enhances the versatility of the new plasmids and their availability for the insertion of cloned DNA. Another distinction is the presence of the DNA segment Tn9 in three of the new hybrid plasmids which provides an additional antibiotic resistance determinant (chloramphenicol). Finally, plasmids pGX1514 and pGX1515 were constructed by ligation of fragments formed by digestion of the starting plasmids with the restriction enzyme AvaI. The hybrid plasmids so constructed have a markedly different composition and orientation of DNA segments from the hybrid plasmids of the literature.

The versatility of the plasmids of this invention allow them to be used in a variety of genetic engineering techniques. As stated, they are useful for cloning procaryotic and eucaryotic genes in a range of microorganisms such as Pseudomonas, E.coli, Rhizobium, Salmonella, and Rhodopseudomonas. They also can be used as shuttle vectors between these species. Genes often can be cloned conveniently in E.coli, then transferred to a variety of organisms. Finally, because these plasmids are based on pBR322 they are useful in various subcloning procedures. Specific genes may be cloned in special purpose vectors, many of which are pBR322 derivatives. The cloned genes then can be cloned into the vectors of this invention and moved into microorganisms.

The present invention is further illustrated by the following examples, which are not intended to be construed as limiting. The procedures and techniques employed in the examples for digestions, ligations, transformations and the like were all conventional and are well known to skilled molecular biologists. Therefore, certain experimental details have been omitted for clarity and brevity.

## Example I

Bacterial strains and media. Bacterial strains used were

Escherichia coli HB101 (Boyer, H.W., and D. Roulland-Dussoix, J. Mol. Biol. 41:459:472, 1969),

Escherichia coli MG1655 (Guyer, M.S., R.R. Reed, J.A. Steitz, and K.B. Low, Symp. Quant. Biol., v. XLV, pp. 135-140, 1981),

Pseudomonas aeruginosa PAO1161 (Dunn, N.W. and B.W. Holloway, Genet. Res. Camb. 18: 185-197, 1981),

Pseudomonas putida KT2440 (Bagdasarian, M., et al. Gene 16:237-247 (1981)).

Plasmids pBR322 (Bolivar, F., et al., Gene 2:95-113, 1977), RSF1010 (Guerry, P., J. Van Embden, and S. Falkow, J. Bacteriol 117: 619-630, 1974), and pMS2 which is colE1 containing Tn9 (Rosner, J.L. and M.M. Gottesman. DNA

Insertion Elements, Plasmids and Episomes, pp. 213-218 (Bukhari, A.L.; Shapiro, J.A. and Adhya, S.L., eds.) Cold Spring Harbor Laboratories, Cold Spring Harbor, New York (1977)) were prepared from cell extracts of E.coli HB101 as follows: Five ml of an early stationary phase cell culture grown in 20 ml M9 minimal medium (Miller, J.H., Experiments in Molecular Genetics, Cold Spring Harbor Laboratory, Cold Spring Harbor, New York, 1972) plus required supplements was subcultured to 500 ml of the same medium and incubated overnight at 37°C. Plasmid pBR322 was amplified by addition of 9 ml chloramphenicol (10 mg/ml in 15% ethanol) to the culture when it reached an $O.D._{540}=0.4-0.5$. Cells were sedimented (16,000 x g, 10 minutes, 5°C.), washed once with 10mM Tris-HCl (pH 8.0) containing 1mM EDTA, suspended in 8 ml 25% sucrose-50mM Tris-HCl (pH 8.0) and transferred to 30 ml centrifuge tubes. To the cell suspension was added 1.5 ml lysozyme (5mg/ml in 0.25 M Tris-HCl, pH 8.0) and 3.4 ml of 0.25 M EDTA. Cells were incubated for 5 minutes on ice, then 15 ml lysing solution (50 mM Tris-HCl, pH 8.0, 10% Triton X-100, 62.5 mM EDTA) were added, and cells were lysed 10 minutes at 22°C. The cell extracts were centrifuged (60,000 x g, 2 hours, 5°C.) and the supernatant collected. Ribonuclease (DNase-free) was added to 10 ug/ml and the extract incubated 30 minutes at 37°C.

Purifications. Closed circular plasmid DNA was purified by cesium chloride/ethidium bromide density gradient ultracentrifugation, essentially as described (Maniatis, T., E.F. Fritsch, and J. Sambrook, Molecular Cloning, Cold Spring Harbor Laboratory, Cold Spring Harbor, New York, pp. 93-94, 1982), except that tubes were centrifuged 15 hours (190,000 x g, 20°C.) in a VTi50 vertical rotor.

## Example II

Transformations.  Competent bacterial cells were used for plasmid transformations.  A 0.5 ml inoculum from a cell culture grown overnight in L broth (Miller, J.H., Experiments in Molecular Genetics, Cold Spring Harbor laboratory, Cold Spring Harbor, New York, 1972) was added to 50 ml prewarmed L broth and incubated at 37°C. with aeration until $O.D._{650}=0.2$.  The culture was chilled on ice for 20 minutes, then cells were sedimented (6,000 x g, 10 minutes, 4°C.) and washed with 25 ml ice-cold 100 mM $MgCl_2$.  Cells were sedimented, suspended in 25 ml ice-cold 100mM $CaCl_2$-50 mM $MgCl_2$ and tubes were placed on ice for 1 hour.  Cells were sedimented, suspended in 0.5 ml ice-cold 100mM $CaCl_2$-50mM $MgCl_2$ and tubes were placed on ice for 1 hour.  Competent cells were used immediately or stored in 30% glycerol at -80°C.

In transformations, 10 ul transforming DNA were added to 0.1 ml competent cells, which were incubated on ice for 20 minutes and then heat-pulsed at 42°C. for 3 minutes. The tubes were placed on ice, 1.5 ml L broth were added, and the cells were incubated with aeration at 37°C. for 90 minutes.  Aliquots of the transformation mixture were plated onto selective media.

Hereinafter, the above procedures will be referred to only by name.

## Example III

Plasmid Constructions.  Plasmids were constructed by restriction enzyme digestion and ligation of specific fragments.  Digestion and ligation reactions were carried out in accordance with conditions specified by the enzyme manufacturers (Bethesda Research Laboratories, Inc. and New England Biolabs, Inc.) unless otherwise noted.

## A. Construction of Plasmid pGX216

Plasmid RSF1010 (1µg) was digested with EcoRI, phenol extracted, ethanol precipitated, and dissolved in 33 µl $H_2O$. The EcoRI-generated staggered ends were filled by treatment with DNA polymerase I in the presence of dATP and dTTP. The reaction mixture contained 100µM dATP, 100 µM dTTP, 1.0mM 2-mercaptoethanol, 67 mM Tris-HCl (pH 7.4), 7.5 mM $MgCl_2$, and 10 units DNA polymerase I. The mixture was incubated 75 minutes at 22°C. The plasmid DNA was phenol extracted, ethanol precipitated, suspended in 17 µl $H_2O$, and ligated with $T_4$ DNA ligase (New England Biolabs), according to manufacturer's instructions. The ligation mixture was digested again with EcoRI to linearize EcoRI sensitive plasmids and the mixture was transformed into E.coli MG1655, selecting $Sm^r$ (50µg/ml). Plasmid DNA, designated pGX214, was purified from one of the transformants that contained an EcoRI-resistant plasmid.

Plasmid pMS2 (1µg) was partially digested with 0.5 units BalI at 37°C. for 5 minutes, and pGX214 was digested with PvuII. The digested plasmids were mixed, ligated, and transformed into E.coli MG1655, selecting $Sm^r$ (50 µg/ml) and $Cm^r$ (12.5 µg/ml). Transformants were obtained, and one isolate containing a 10.3 kb plasmid, designated pGX216, was identified. Plasmid DNA was purified and characterized by restriction enzyme mapping.

## B. Construction of Plasmids pGX1509 and pGX1510

Plasmids pBR322 and pGX216 were digested with PstI (New England Biolabs) for 2 hours at 37°C. Equimolar concentrations of the fragments from the two plasmids were mixed and ligated at 16°C overnight. The ligation mixture was transformed into E.coli HB101, selecting $Tc^r$ (25 µg/ml). Transformants were scored for $Cm^r Sm^s$ and two isolates were identified. Plasmids in these strains, designated pGX1509 and pGX1510, were characterized, and the orientation of pBR322 determined, by restriction analysis.

C.    Construction of Plasmids pGX1514 and pGX1515

RSF1010 and pBR322 were each digested with AvaI (Bethesda Research Laboratories) for 2 hours at 37°C. and equimolar concentrations of the fragments were mixed and ligated at 16°C. overnight. The ligation mixture was used to directly transform competent cells of P.aeruginosa PAO1161, selecting $Tc^r$ (100 µg/ml). Two of the transformants contained 9.0 kb plasmids, which were designated pGX1514 and pGX1515. These plasmids were purified, transformed into E.coli HB101, repurified, and characterized by restriction enzyme analysis. Digestion of pGX1514 and pGX1515 with AvaI and separation of fragments by agarose gel electrophoresis indicated that both plasmids contained the 3.0 and 1.7 kb AvaI fragments of RSF1010. Digestion with PvuII indicated that the orientation of the 1.7 kb AvaI fragment in pGX1514 is different than in pGX1515 (see Fig. 2).

### Example IV

Determination of host range. The new plasmids were transformed into Pseudomonas as an indicator that the hybrid plasmids confer the wide host range property of RSF1010. Plasmids pGX216, pGX1509, and pGX1510 were prepared from E.coli HB101 or MG1655 and were transformed into competent cells of P.aeruginosa PAO1161, selecting $Cm^r$ (1 mg/ml) or $Tc^r$ (100 µg/ml). Plasmids pGX1509 and pGX1510 were purified from PAO1161 and used to transform competent cells of P.putida KT2440, selecting $Tc^r$ (100 µg/ml). In each case, transformants were isolated that contained intact plasmid DNA of the appropriate size. Plasmids pGX1514 and pGX1515 were isolated in Pseudomonas and were transformed into E.coli HB101.

## Claims

1. A novel hybrid plasmid consisting of DNA from plasmid RF 1010 together with the DNA of the transposon Tn 9 and/or the DNA of plasmid pBR 322, the DNA from plasmid RF 1010 lacking at least the EcoRI restriction site of the original plasmid.

2. A microorganism transformed by the plasmid of claim 1.

3. The microorganism of claim 2 of the genus Escherichia.

4. The microorganism of claim 3 of the species coli.

5. The hybrid plasmid pGX1509.

6. A microorganism transformed by the plasmid of claim 5.

7. The microorganism of claim 6 of the genus Escherichia.

8. The microorganism of claim 7 of the species coli.

9. A microorganism of the genus and species E.coli designated as GX3601 (pGX1509) and deposited with the Northern Regional Research Laboratory as NRRL B-15328.

10. The hybrid plasmid pGX1510.

11. A microorganism transformed by the plasmid of claim 10.

12. The microorganism of claim 11 of the genus Escherichia.

13. The microorganism of claim 12 of the species coli.

14. A microorganism of the genus and species E.coli designated as GX3602 (pGX1510) and deposited with the Northern Regional Research Laboratory as NRRL B-15329.

15. The hybrid plasmid pGX1514.

16. A microorganism transformed by the plasmid of claim 11.

17. The microorganism of claim 16 of the genus Escherichia.

18. The microorganism of claim 17 of the species coli.

19. A microorganism of the genus and species E.coli designated as GX3639 (pGX1514) and deposited with the Northern Regional Research Laboratory as NRRL B-15331.

20. The hybrid plasmid pGX1515.

21. A microorganism transformed by the plasmid of claim 16.

22. The microorganism of claim 21 of the genus Escherichia.

23. The microorganism of claim 22 of the species coli.

24. A microorganism of the genus and species E.coli designated as GX3640 (pGX1515) and deposited with the Northern Regional Research Laboratory as NRRL B-15332.

25. The hybrid plasmid pGX216.

26. A microorganism transformed by the plasmid of claim 25.

27. The microorganism of claim 26 of the genus Escherichia.

28. The microorganism of claim 27 of the species coli.

29. A microorganism of the genus and species E.coli designated as GX3607 (pGX216) and deposited with the Northern Regional Research Laboratory as NRRL B-15330.

FIG I

FIG. 2